# EUROPEAN PATENT SPECIFICATION

(11) **EP 3 994 984 B1**
(45) Date of publication and mention of the grant of the patent: **24.05.2023**
(21) Application number: 21206744.1
(22) Date of filing: 05.11.2021
(51) Int. Cl.: C12N 5/077, C12N 5/00, A01N 41/10, A01N 43/16, A01P 1/00, A01N 1/02

(54) **A METHOD FOR THE CRYOPRESERVATION OF SAMPLES OF ADIPOSE TISSUE**
VERFAHREN ZUR KRYOKONSERVIERUNG VON FETTGEWEBE
PROCEDÉ DE CRYOCONSERVATION DE TISSU ADIPEUX

(30) Priority: 05.11.2020 IT 202000026467
(43) Date of publication of application: 11.05.2022
(73) Proprietor: Lipobank S.r.l., 20129 Milano (IT)
(72) Inventor: DI FEDE, Sergio, 40100 Bologna (IT); VENTURA, Carlo, 40100 Bologna (IT)
(74) Representative: Soldatini, Andrea

(56) References cited:
- WO-A1-98/14058
- CN-A- 105 052 894
- US-A- 5 827 741
- ZHIQUAN SHU ET AL: "Update on Cryopreservation of Adipose Tissue and Adipose-derived Stem Cells", CLINICS IN PLASTIC SURGERY., vol. 42, no. 2, 1 April 2015 (2015-04-01), pages 209-218, XP055518136, GB ISSN: 0094-1298, DOI: 10.1016/j.cps.2014.12.001

## Description

### Field of the invention

The present invention relates generally to the field of biological sample processing, and more specifically to a method for the cryopreservation and thawing of human adipose tissue samples, useful in cosmetic surgery and regenerative medicine; and to a kit for carrying out such a method.

### State of the Art

In recent years, it has become increasingly popular in cosmetic and reconstructive plastic surgery, as well as in the treatment of more complex conditions, such as osteoarticular lesions, peripheral vasculopathies, pressure ulcers, anal incontinence and anorectal fistulas, to use human adipose tissue derivatives obtained by non-enzymatic mechanical processing, according to "minimal handling" procedures (in accordance with the criteria established by the European Medicines Agency - EMA), which do not involve the use of enzymes, forced centrifugation and cell separation on a gradient. The advantage of such adipose tissue derivatives is manifold compared to systems for isolation and subsequent expansion (*in vitro* culture) of adipose-derived stem cells (ASCs). In fact, the tissue derivatives mentioned above:
- They do not involve the enzymatic digestion of adipose tissue, nor the isolation and subsequent culture of ASCs *in vitro* in dedicated cell factories approved for cell/tissue processing according to Good Manufacturing Practice (GMP) criteria.
- They maintain the integrity of the vascular-stromal niche, which is the natural microenvironment in which the pericytes, the precursors of ASCs, reside in *vivo* and provides the latter with multiple chemical and physical signals that are essential for the performance of the regenerative properties of the stem component itself.
- They require extremely short processing times, allowing for immediate clinical use.
- They do not have the regulatory restrictions associated with enzymatic processing of adipose tissue and subsequent expansion of ASCs in culture.
- They do not involve the risk of senescence of ASCs, as the tissue derivative is transplanted for autologous use within minutes of harvesting the adipose tissue by liposuction. In contrast, classical methods of enzymatic digestion and subsequent expansion of ASCs in dedicated environments (GMP Cell Factories) involve prolonged culture times that are associated with the development of cellular senescence with consequent loss of stem regenerative potential.

For these reasons, in the last decade an increasing number of devices and methods have been developed and made available on the market to realise non-enzymatic mechanical processing systems of human adipose tissue that could be subject to autologous transplantation for the purpose of reconstructive plastic, aesthetic surgery and more complex Regenerative Medicine approaches.

Due to the good results achieved so far in a wide variety of areas, this approach is becoming increasingly popular. However, it is now clear that the clinical conditions for which transplantation of these tissue derivatives is performed often require more than one treatment over time. This involves performing a new liposuction for each repetition of the treatment, which is particularly inconvenient for the patient. In addition, if one considers that only a small part of the tissue derivative, often less than 50% of the volume, is used for transplantation with each treatment, it follows that a substantial amount of valuable biological material becomes in fact an unusable waste product.

Therefore, the availability of a method capable of guaranteeing not only the cryopreservation of unused quantities of adipose tissue derivative, but also and above all a thawing dynamic that maintains the vitality and structural properties of the preparation unaltered, so as to make them comparable to those of the fresh preparation, would have a considerable impact on the use and spread of modern strategies of Regenerative and Precision Medicine. Such a method would not only dramatically reduce the inconvenience of repeating the liposuction procedure but would also allow operators to plan and carry out targeted procedures and treatments with extreme flexibility, significantly reducing the time needed to carry out each procedure and eliminating the need to repeat liposuction. In addition, according to currently available data on cryopreservation of different types of stem cells, the tissue derivative could maintain its biological characteristics unchanged over the time of cryopreservation, being able to show its regenerative potential even after many years in the same patient.

The availability of such a method could therefore optimise current treatments for age-related imperfections, such as those of the face and hands, or those related to reduced subcutaneous adipose tissue. The same method could also be used to optimise the treatment of more serious and complex problems, such as those related to wound-healing, peripheral vasculopathies (e.g. diabetic foot, atherosclerotic skin ulcers, pressure ulcers), osteoarticular and tendon injuries, incontinence and anal fistulas, all problems for which there is a steady increase in the practice of autologous transplantation of adipose tissue derivatives obtained by non-enzymatic mechanical processing methods. In particular, it has been scientifically demonstrated that autologous transplantation of these derivatives is also used in orthopaedics to speed up recovery from sports injuries, to treat arthritis and tendinopathies, and in gynaecology to treat vaginal atrophy and post-menopausal urinary incontinence. Indeed, it has been known for several years that human adipose tissue is a source of multipotent mesenchymal stem cells (ASCs) that can differentiate into various cell types, including osteocytes, adipocytes, neural cells, vascular endothelial cells, cardiomyocytes, hepatocytes, etc., and thus have strong therapeutic potential in various fields of regenerative medicine.

The first attempts to preserve adipose tissue, dating back to the last decade, were directed towards refrigerating the material or freezing it at relatively high temperatures comprised between +1°C and -18°C for a relatively short time. However, these systems and in particular simple refrigeration, even for short periods, have proved unsuitable for preserving viable tissue.

Subsequent studies have focused on freezing at very low temperatures with liquid nitrogen, which has also shown much better results in terms of maintaining mitochondrial metabolic activity, but always for relatively short freezing periods in the order of days.

A further decisive improvement was made by adding a cryoprotective agent to the adipose tissue to be frozen, then carrying out the freezing procedures in a controlled manner, to prevent intracellular ice formation during cooling and at the same time minimise cell damage due to too slow cooling. Zhiquan Shu published in "Clinics in Plastic Surgery, vol. 42, no.2, 209-218, (2015)" a study on the cryopreservation and use in cell-therapy and tissue engineering of adipose tissue or adipose derived stem cells. The study describes the advantages of cryopreserving in the presence of DMSO and trehalose and the necessity of removing DMSO when the cells are brought to higher temperatures.

According to a protocol now recognised as optimal for preserving the viability of adipose tissue and other biological tissue samples over long periods of time by freezing, a mixture of dimethyl sulfoxide (DMSO) and trehalose, added to the tissue before freezing, proved to be the ideal cryoprotective agent. According to this protocol, freezing is then achieved starting from room temperature down to -30°C with a programmed cooling of 1-2°C per minute, before immersing the sample in liquid nitrogen where the temperature reaches approximately -195°C, while thawing is carried out by simply taking the sample out of the liquid nitrogen and bringing it to room temperature, possibly immersing it in water at 37°C until it is completely thawed.

Adipose tissues stored according to this protocol, after thawing, have the same appearance as freshly harvested tissues; however, their quality has been found to be inferior to that of fresh tissues in terms of cell viability and histology, although there is an improvement with respect to tissues frozen without a cryoprotective agent. See Lee L.Q. Pu Organogenesis (2009) 5:3, 138-142.

More recently, studies have also been published proposing adipose tissue cryopreservation protocols that do not use cryoprotective agents but claim to achieve improved tissue viability after storage by modulating the freezing temperature. Zheng W. et al. in Aesth Plast Surg (2019) 43:826-835 argue, for example, that cooling lipoaspirates to -80°C without addition of cryoprotective agents has an effect of better maintaining cell morphology, their adhesive and proliferative properties, compared to storage at higher temperatures and also at liquid nitrogen temperature. However, the authors indicate that even at this optimum freezing temperature the material obtained is damaged, shows fibrosis and a not optimal degree of resorption. Also in this study, thawing of the material is simply carried out by immersion of the tissue in water at 37°C, and different thawing conditions and their consequences on the viability of the material obtained are not investigated.

In the light of the above discussion on the clinical applications of adipose tissue derivatives and the prospects offered by their use even long after liposuction surgery, it is clear that there is still much felt the need for new methods of storing and thawing these tissues that maintain their vitality and regenerative properties.

### Summary of the invention

The Applicant has now developed a method of cryopreservation of human adipose tissue samples, in particular micro-fragmented adipose tissue, which solves the problems described above for the known methods by using a special thawing protocol, and has the characteristics described in detail below.

In particular, the method of this invention makes it possible to obtain tissues with improved cell viability after long-term storage in the frozen state, compared with tissues obtainable by known methods.

At the same time, the method of this invention achieves the aforementioned result in a simple manner, with readily available reagents and a user-friendly kit which is also a subject of this invention.

The subject of the invention is therefore a method for the cryopreservation of a sample of adipose tissue previously taken from a subject, whose essential features are defined in the first of the appended claims.

A further subject of the invention is a kit for carrying out the above method, whose essential features are defined by the respective independent claim appended hereto.

A further subject of the invention is the use of the above-mentioned kit for thawing samples of adipose tissue coming from cryopreservation for transplantation in cosmetic surgery and regenerative medicine, whose essential features are defined in the appended independent claim.

Other important features of the cryopreservation method, of the kit for making it and of its use, according to the invention, are set out in the following detailed description and defined in the appended dependent claims.

### Brief Description of the Figures

Figure 1: fluorescence light microscope image of a fresh human adipose tissue sample, just processed with a known minimal mechanical handling device called Lipogems^{®}, then treated with the Thermofisher LIVE/DEAD^{™} cell viability assay kit, as described in the following experimental part;
Figure 2: fluorescence light microscope images of 4 separate human adipose tissue samples micro-fragmented with the Lipogems^{®} device, thawed after cryopreservation according to the method of this invention and treated with the Thermofisher LIVE/DEAD^{™} cell viability assay kit, as described in the following experimental part;
Figure 3 (state of the art): fluorescence light microscope image of a human adipose tissue sample micro-fragmented with the Lipogems^{®} device, thawed after cryopreservation by a conventional thawing method, and treated with the Thermofisher LIVE/DEAD^{™} cell viability assay kit, as described in the following experimental part;
Figure 4: fluorescence light microscope images of a fresh human adipose tissue sample micro-fragmented with the Lipogems^{®} device, after fixation (a); and of the same sample thawed after cryopreservation by the method of this invention (b), as described in the following experimental part;
Figure 5: embodiment of a kit for carrying out the thawing step of the method of the invention, wherein the containers for the adipose tissue sample comprise bags and the different thawing solutions used in the present method are contained in syringes, everything included in a closed circuit.

### Detailed description of the invention

In the context of this invention, "human adipose tissue sample" means a sample of adipose tissue previously taken from a human subject, preferably a microfragmented human adipose tissue (or MAT) sample. Micro-fragmented adipose tissue is a known derivative of the adipose tissue, obtained by mechanical, non-enzymatic processing of this type of tissue; it is composed of several cell types, including adipocytes, pericytes and mesenchymal stem cells within a preserved vascular-stromal niche. Non-limiting examples of minimally manipulative processes for obtaining microfragmented adipose tissue from lipoaspirated tissue are described for example by Veronese S. et al. in Journal of Tissue Engineering and Regenerative Medicine, 2020, 14, 1213-1226; the processes described herein consist of centrifugation at low gravity acceleration (between 1000 and 400 rpm for less than 5 minutes), emulsification, filtration, fragmentation, pureeing, sedimentation, squeezing, washing in physiological saline solution or Ringer's lactate solution, and combinations thereof.

Micro-fragmented human adipose tissue was obtained with the Lipogems^{®} device, as described in Bianchi F. et al. Cell Transplant, 2012, 22, 2063-2077. In short, this disposable device, already approved by both the FDA and the EMA, consists of a plastic cylinder containing two stainless steel filters at its ends, together with 5 stainless steel spheres, capable of progressively micro-fragmenting the liposuctioned tissue up to clusters of 300-400 µm in diameter, together with a washing thereof during processing, which guarantees the elimination of oil residues from the liposuctioned tissue (thus eliminating the associated pro-inflammatory risk), as well as the elimination of cell and tissue debris from the initial liposuction process.

"Physiological saline solution" in the present invention means a saline solution, for example a sodium chloride solution, in water for injectable preparations, i.e. in sterile water; typically, such a sodium chloride solution has a concentration of 0.9% w/v.

The method for the cryopreservation of an adipose tissue sample of this invention comprises a step of freezing the tissue, a step of storing the frozen tissue for a specified period of time, and a step of thawing the tissue, wherein the thawing step is carried out by successive washes of the tissue with solutions of trehalose and dimethyl sulfoxide (DMSO) at decreasing concentrations down to 0 of DMSO.

The inventors found that this particular thawing protocol preserves the integrity of the tissue sample to a greater extent than the traditional thawing method, which simply involves immersing the container in water at 37°C and washing it with water. Compared to conventional thawing protocols, that of the invention does not complicate the procedures, as it only involves washing; the last wash with trehalose solution, without DMSO, also allows the DMSO to be completely removed before tissue transplantation.

In a preferred embodiment, the thawing step of the method of this invention comprises at least two, and preferably at least three, successive washes of the tissue with solutions of decreasing concentration of DMSO, the last wash being carried out with a trehalose solution having a 0 concentration of DMSO. Preferably, washing solutions with a decreasing concentration of DMSO, all have the same concentration of trehalose, advantageously a concentration of 0.2 M which was optimal in terms of cell viability obtained after thawing. According to the invention, the solutions used may for example have a trehalose concentration comprised between 0.05 M and 0.4 M, and preferably between 0.1 M and 0.3 M. Also, the concentration of DMSO in the solutions used in the method of the invention, which decreases to 0 from the first solution used to the last solution containing only trehalose, may for example be comprised between 0.05 M and 0.4 M, and preferably between 0.1 M and 0.3 M.

In an aspect, the tissue freezing step is carried out in a controlled blast chilling device, with gradual lowering of the temperature from room temperature, e.g. by lowering the temperature by 1-3°C per minute. Once a temperature of approximately -180°C has been reached, the tissue is preferably transferred into liquid nitrogen, so as to reach a temperature of approximately -195°C, at which the tissue can be stored for periods of time typically ranging from about 2 weeks to about 5 years.

Before freezing, a known cryoprotective agent, e.g. an aqueous solution of trehalose and DMSO, can be added to the tissue. In a preferred aspect, this solution is added drop by drop to the adipose tissue prior to blast chilling, directly into the cryopreservation container, e.g. in a 1:1 volume ratio.

The method of this invention may be implemented by means of a kit comprising several containers suitable for housing the adipose tissue sample in the thawing phase for its washing, for example ethylene vinyl acetate (EVA) bags, test tubes or laboratory tubes, and several ready-to-use washing solutions of trehalose and DMSO, at decreasing concentrations of DMSO down to 0. This kit, which is also part of this invention, can be used to thaw samples of adipose tissue from cryopreservation and intended for transplantation in cosmetic surgery and regenerative medicine, in particular samples of human adipose tissue from cryopreservation for lipofilling operations with autologous tissue transplantation.

As explained above and illustrated by the experimental data provided below, the main advantage of the method for cryopreservation of adipose tissue samples of this invention lies in the fact that the thawed tissue has been shown to have greater viability than tissue treated by known methods. This clearly represents a great progress since only viable tissue can be transplanted and used in cosmetic surgery and regenerative medicine. Tissues with greater viability also showed a lower degree of resorption by surrounding tissues, thus removing the time required for a possible repeat transplant.

A further advantage of this invention is that it is a simple method that can be easily adopted in any clinic, using simple-to-use reagents and a ready-to-use kit of reagents and containers.

A further advantage of the present method is that the washes involved in the innovative thawing protocol of the invention facilitate the extraction of the tissue from the container in which it was stored in frozen form.

The present invention therefore represents an optimal solution from every point of view, and overcomes the drawbacks highlighted above in describing the state of the art.

The following examples are provided for the purpose of illustrating the present invention but do not constitute a limitation thereof.

### Experimental part

### Materials and methods used

The samples of human adipose tissue used by the inventors in the following experiments are samples taken from patients of the Istituto Ortopedico Rizzoli, Via Giulio Cesare Pupilli, 1 40136 Bologna, Italy, subject to their free and informed written consent for the collection and within the framework of a clinical study carried out on the material collected, and described below, which has also been approved by the Ethical Committee of the Istituti Ortopedici Rizzoli. The samples were not stored but destroyed after analysis.

The LIVE/DEAD^{™} kit used in the cell viability assay in the following examples is the mammalian cell kit sold by ThermoFisher under catalogue number L3224. All other reagents and culture media used in the following are sold by Sigma Aldrich.

### EXAMPLE 1 - Cryopreservation of a human adipose tissue sample

Approximately 30 minutes before freezing, a solution of cryoprotective agent (0.5 M in DMSO and 0.2 M in trehalose) was prepared from the sterile stock solutions of DMSO (1 M) and trehalose (0.4 M). The solution was kept at a temperature of 4°C until use.

To a 15 ml test tube containing a sample of fresh adipose tissue, processed with Lipogems^{®}, the cryoprotective solution prepared as above was added drop by drop, in a 1:1 ratio, and transferred into 0.5 ml sealed, cryopreservation-specific paillettes.

Using a controlled blast chiller, the paillettes were subjected to slow and controlled freezing, lowering the temperature by 1°C per minute to -18°C, then they were transferred into liquid nitrogen at a temperature of approximately -195°C.

### EXAMPLE 2 - Sample thawing by the method of the invention

After a period of time of 15 days in liquid nitrogen, part of the adipose tissue samples frozen as described above in Example 1 were prepared for thawing according to the protocol of the invention in the following manner.

Approximately 30 minutes before thawing the samples, the following solutions of decreasing concentration of DMSO were prepared, each with a volume of 6 ml), by mixing solutions of trehalose in physiological saline solution and DMSO in physiological saline solution, at the concentrations indicated below:
Solution 1: 0.3 M of DMSO + 0.2 M of trehalose
Solution 2: 0.1 M of DMSO + 0.2 M of trehalose
Solution 3: 0.2 M of trehalose

The paillette-type cryopreservation containers were then removed from the liquid nitrogen and taken to an area at room temperature, where the ends of the containers were cut to release the frozen tissue and transfer it to a sterilised 15 ml collection tube. The release of the tissue was facilitated by running solution 1 through the container and pouring it into the collection tube together with the tissue.

Using sterile Pasteur pipettes, the adipose tissue was then transferred from the collection tube containing solution 1 from the first wash into a second tube containing solution 2, where the tissue continued to be gently mixed in the solution before being transferred to a third tube containing solution 3 where it was again gently agitated.

Finally, adipose tissue from the third tube, where solution 3 remained, was transferred to a tube containing 6 ml of complete cell culture medium consisting of Dulbecco's Modified Eagle Medium/Low Glucose, 1% L-Glutamine, 1% penicillin-streptomycin, and 20% Fetal Bovine Serum. After an overnight incubation at 37°C with 5% CO₂, the adipose tissue was subjected to the following viability analyses, metabolic activity and evaluation of the architecture of the vascular-stromal niche.

### Cell viability assay

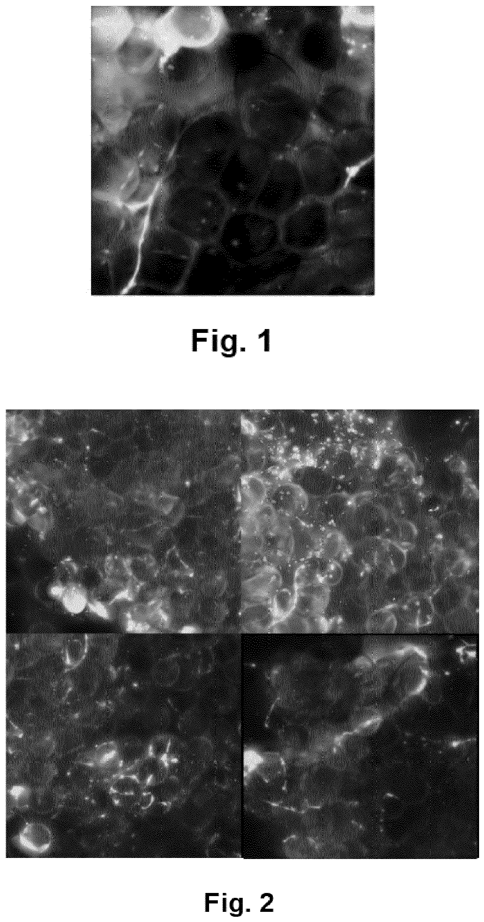

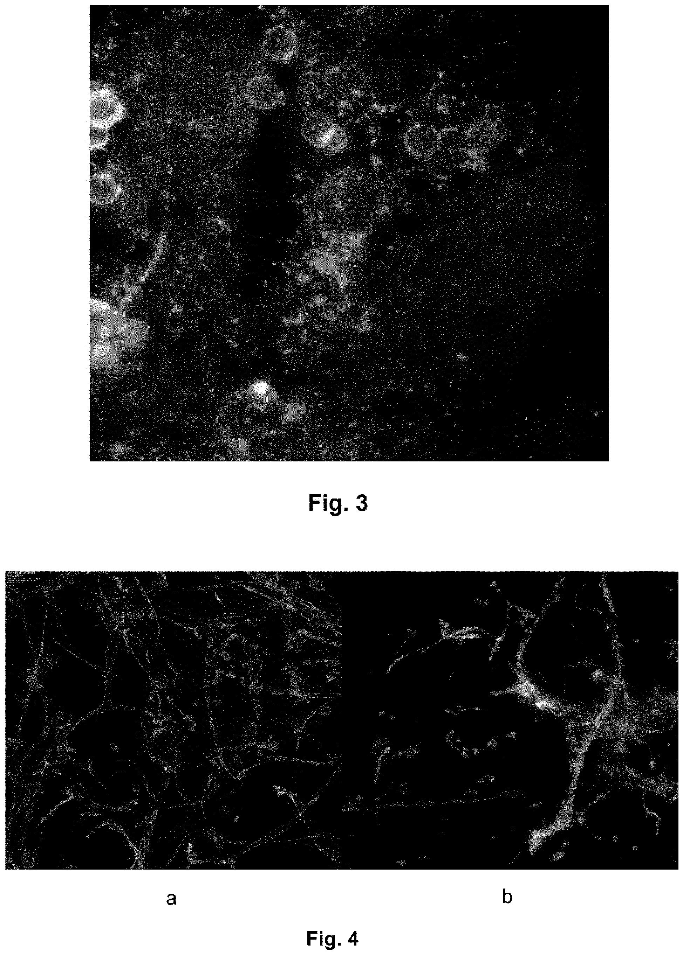

The cell viability assay on thawed tissue as described above was conducted with a LIVE/DEAD^{™} kit for mammalian cells sold by ThermoFisher, catalogue number L3224, using a Nikon Inverted Microscope Eclipse Ti-E light microscope (Nikon Instruments, Tokyo, Japan) equipped with a DS-03 digital camera connected to a computer and operated by NIS-Elements Imaging Software, version 4.30 (Nikon Instruments). The images in Figures 1 and 2 were thus recorded. While Figure 1 shows the recorded image for the fresh adipose tissue sample, just collected, Figure 2 shows images for the cryopreserved and thawed sample according to the protocol of the invention as described above. In both cases, the massive presence of the intact membrane rhyme of the adipocyte cells (the largest in the figure, and round in shape) is clear, a sign of the quality of preservation of the sample, highlighted in the colour images by the green colour. In fact, adipocytes are "taut" and their membrane is very thin, unlike that of other cells, which are more compact. Therefore, if they were damaged during thawing, they would lose their membrane integrity, which in this case is intact. Red in the colour images show the nuclei of cells whose membrane is no longer intact, so the propidium iodide reagent in the kit reaches the DNA in the nucleus and colours it in red. The inventors were able to observe that this phenomenon affects a very small percentage of cells, less than 10%. Moreover, this does not mean that these cells are actually dead; it is plausible that the dye passes through micro-holes in cells that are still viable in morpho-functional recovery. To confirm this observation, Figure 3 shows the image recorded for a human adipose tissue derivative in which membrane integrity was severely compromised during thawing carried out using a standard protocol different from that of the invention and described below in Example 3. The inventors were able to observe in this case, as explained in more detail below, that only a few adipocytes were intact, with only one large vessel remaining compact and viable, for that matter, for a short section, and in any case surrounded by a large number of altered, possibly dead, pericitary cells.

### Metabolic activity assay (MTT Test)

The MTT assay is a standard colorimetric assay for measuring the activity of enzymes that reduce 3-[4,5-dimethylthiazol-2-yl]-2,5-diphenyl tetrazolium bromide, or MTT, which is yellow, to formazan, which is a blue/purple colour. The mitochondrial reductase enzyme, succinate dehydrogenase, is in fact only active in living cells and its function is to break the tetrazolium ring of MTT (yellow) with the formation of formazan (blue). This reaction can be easily assessed and measured by spectrophotometric reading of the sample at a wavelength of 570 nm. The MTT assay is therefore used to determine the cytotoxicity of drugs or other types of substances/treatments in cell cultures. This assay is generally carried out on adherent cells, which are then dissolved so as to liquefy the crystals metabolised over time by the cells. The amount of colour in the mixture in relation to the number of cells gives an indication of the amount of metabolic activity in the culture itself.

In the assay carried out by the inventors, the adipose tissue under examination was cut intact, the assay was not carried out on single cells in culture, so by its nature it is not solubilised due to the massive presence of fatty acids, extracellular matrix, etc., and therefore the reaction cannot be quantified by the standard method described above. However, the inventors observed that the entire sample of treated adipose tissue, i.e. 100% of the treated area, immediately took on a deep purple colour, with clear presence of crystals over the entire depth of the sample, indicating complete metabolic activity of the cells in the adipose tissue thawed by the method of the invention.

### Evaluation of the vasculo-stromal niche

A just harvested sample of fresh human adipose tissue, and a sample of the same tissue subjected to cryopreservation for 15 days in liquid nitrogen and subsequent thawing by the method of the present invention, were fixed and incubated with monoclonal antibodies directed to (VE)-cadherin, an adhesion molecule very important in maintaining the integrity and permeability characteristics of vascular endothelium, and to CD31 (PECAM-1), an essential marker of endothelial cells that is used to identify these cells and track their distribution. These markings, which give a red and a green colour respectively, make it possible to appreciate the structure of the so-called vascular-stromal niche, i.e. the sort of special topography in which the stem elements reside, in the context of specific vascular architectures.

The inventors, with this experiment, were able to appreciate the total persistence of the integrity of the vascular-stromal niche, which was in fact indistinguishable in the sample thawed by the method of this invention (Figure 4 b) compared to the fresh tissue sample (Figure 4 a). This is due to the very high vitality found in samples thawed by the method of the present invention (see Figure 2). In other words, the thawing protocol becomes fundamental so that the structural integrity of the vascular-stromal niche is associated with the functional integrity of its component elements.

### EXAMPLE 3 - Sample thawing according to conventional procedure

After a period of 15 days in liquid nitrogen, part of the adipose tissue samples frozen as described above in Example 1 were thawed according to the protocol currently used and described below.

The paillette-type cryopreservation containers were then removed from the liquid nitrogen and taken to an area at room temperature, where the ends of the containers were cut to release the frozen tissue and transfer it to a sterilised 15 ml collection tube containing physiological saline solution. Using a Pasteur pipette, the solution was gently stirred in the tube, then the tissue was transferred to a tube containing 6 ml of complete cell culture medium consisting of Dulbecco's Modified Eagle Medium/Low Glucose, 1% L-Glutamine, 1% penicillin-streptomycin, and 20% Fetal Bovine Serum. After an overnight incubation at 37°C with 5% CO₂, the adipose tissue was subjected to viability analysis with the same kit as described above, in parallel with the tissue thawed using the protocol of the invention. The result can be seen in Figure 3, which shows a fluorescence light microscope image of a sample thawed in the state-of-the-art mode: there are only a few green adipocytes, i.e. with the membrane still intact, and only one large vessel appears to have remained compact and viable, for that matter only for a short section. As is clear from the image in Figure 3, these few viable elements are surrounded by a large number of altered, possibly dead, pericitary cells, as highlighted by the red staining of the nucleus.

The MTT assay carried out on thawed adipose tissue confirmed these results, showing only small patches of crystals at specific points in the sample, but only after several hours of culture with the MTT reagent.

### EXAMPLE 4 - Cryopreservation of micro-fragmented adipose tissue by the method of the invention

Approximately 30 minutes before freezing, a solution of cryoprotective agent (0.5 M in DMSO and 0.2 M in trehalose) was prepared from the sterile stock solutions of DMSO (1 M) and trehalose (0.4 M). The solution was kept at a temperature of 4°C until use.

The freezing procedure was performed by transferring 5 ml of adipose sample, previously processed with the Lipogems^{®} device, in a suspension of physiological solution into a large EVA bag, connected by a special tube to a tap. The physiological saline solution still present in the bag was then removed, and 5 ml of the cryopreservative solution, prepared as described above, was added. The sealed bag was then placed inside an EVA cover bag, which was also welded after the air had been sucked out and a vacuum created inside the bag using special equipment.

Using a controlled blast chiller, the tissue derivative contained in each bag was frozen slowly and controlled, lowering the temperature by 1°C per minute to -180°C, and then the bags were transferred to liquid nitrogen at a temperature of approximately -195°C.

### EXAMPLE 5 - Sample thawing by the method of the invention

After a period of time of 15 days in liquid nitrogen, part of the adipose tissue derivative samples frozen as described above in Example 4 were prepared for thawing according to the protocol of the invention in the following manner.

Approximately 30 minutes before thawing the samples, the following solutions of decreasing concentration of DMSO were prepared, each with a volume of 6 ml), by mixing solutions of trehalose in physiological saline solution and DMSO in physiological saline solution, at the concentrations indicated below:
Solution 1: 0.3 M of DMSO + 0.2 M of trehalose
Solution 2: 0.1 M of DMSO + 0.2 M of trehalose
Solution 3: 0.2 M of trehalose

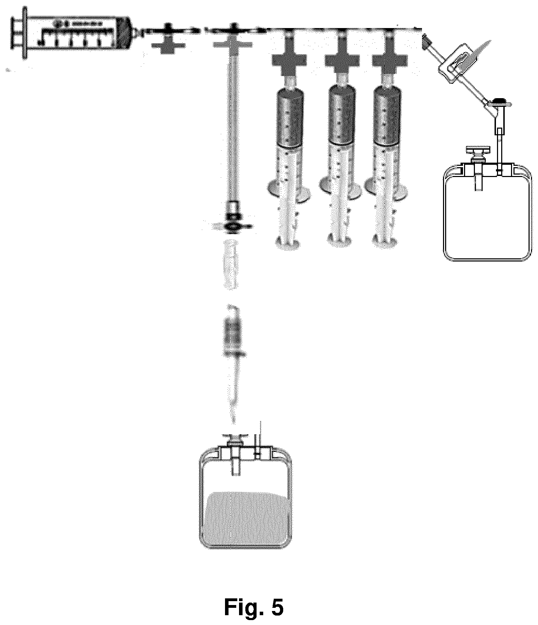

Each solution was placed in 3 syringes from the kit shown in Figure 5.

Thawing is carried out by immersing each bag (small bag of the freezing kit) in a water bath, under a hood at 37°C. The bag is immediately connected to a tube provided with a special "beak" for piercing the septum closing the inlet port described above. This is done by removing the protective cap. The tube is provided with taps in series to allow connection to syringes containing washing solutions. One of the taps is also connected to a sterile filter. During the procedure detailed below, when the washing solution is injected into the bag (the piston is now at full stroke in the syringe), the filter will allow, under absolutely sterile conditions, air to be sucked into the syringe in order to be able to push into the bag any quantities of washing solution that may have remained in the dead space of the tube connected to the bag itself. At this point, the thawing method involves a series of sequential washes. Then, after a few seconds of decanting, the cryopreservation mixture is removed using an empty syringe provided in the kit and connected to the connecting tube to the bag.

A first washing solution is added via a first syringe of the kit, containing 0.3 M DMSO + 0.2 M trehalose. The bag is gently massaged to re-suspend the sample. The first washing solution is removed using a first syringe. The second washing solution containing 0.1 M DMSO + 0.2 M is added immediately via a second syringe, again using the connection tube mentioned above and turning the tap to direct the flow of washing solution into the bag. As above, after re-suspension, the second washing solution is removed using the second syringe; the third washing solution, containing only 0.2 M trehalose, without DMSO, is added immediately. Finally, the third washing solution is removed.

The sample is transferred into a new bag connected to the system, using a new sterile syringe and directing the taps appropriately. The resulting sample is ready for shipment (+4 °C) and further use.

The present invention has been described herein with reference to a preferred embodiment. It is to be understood that there may be other embodiments that relate to the same inventive nucleus, all falling within the scope of protection of the claims provided below.

## Claims

1. A method for the cryopreservation of a sample of human adipose tissue previously taken from a subject, comprising a freezing phase of said tissue, a storage phase of frozen tissue for a certain period of time, and a thawing phase of the tissue, said method being **characterised in that** said thawing phase is carried out by successive washings of said tissue with solutions of trehalose and dimethylsulphoxide (DMSO), at decreasing concentration to zero of DMSO, the last washing being carried out with a solution of trehalose only.

2. The method of claim 1, wherein said biological tissue is micro-fragmented human adipose tissue.

3. The method of claim 1 or 2, wherein said solutions of trehalose and DMSO are solutions in water or in physiological saline solution.

4. The method of any one of the preceding claims, wherein said solutions of trehalose and DMSO have all the same concentration of trehalose and decreasing concentrations to zero of DMSO.

5. The method according to any one of the preceding claims, wherein said concentration of trehalose and said concentration of DMSO ranges from 0.05 M to 0.4 M, preferably from 0.1 M to 0.3 M.

6. The method of any one of the preceding claims, wherein said thawing phase comprises at least three successive washings of said tissue with said solutions of trehalose and DMSO.

7. The method of any one of the preceding claims, wherein said thawing phase comprises three successive washings of said tissue with the following solutions:
- Solution 1: 0.3 M of DMSO + 0.2 M of trehalose
- Solution 2: 0.1 M of DMSO + 0.2 M of trehalose
- Solution 3: 0.2 M of trehalose.

8. The method of any one of the preceding claims, wherein said freezing phase is carried out on said tissue added with a cryoprotective agent, such as a solution of trehalose and DMSO in water or in physiological saline solution.

9. The method of any one of the preceding claims, wherein said freezing phase is carried out by placing the tissue in a suitable container inside a blast chiller having a lowering of temperature programming down to -180°C, then said container is transferred under liquid nitrogen.

10. A kit for carrying out the method of anyone of claims 1-9, comprising more containers capable of hosting said sample of biological tissue in the thawing phase for its washing, and more washing solutions ready for use of trehalose and DMSO, at concentration of DMSO decreasing to zero.

11. Use of the kit of claim 10, for thawing samples of human adipose tissue coming from cryopreservation and intended for transplant in cosmetic surgery and regenerative medicine.

## Patentansprüche

1. Verfahren zur Kryokonservierung einer zuvor einem Probanden entnommenen Probe menschlichen Fettgewebes, enthaltend eine Gefrierphase des Gewebes, eine Lagerphase des gefrorenen Gewebes für einen bestimmten Zeitraum und eine Auftauphase des Gewebes, wobei das Verfahren **dadurch gekennzeichnet ist, dass** die Auftauphase durch aufeinanderfolgende Waschungen des Gewebes mit Lösungen von Trehalose und Dimethylsulfoxid (DMSO) bei abnehmender DMSO-Konzentration zu Null hin durchgeführt wird, wobei die letzte Waschung nur mit einer Lösung von Trehalose durchgeführt wird.

2. Verfahren nach Anspruch 1, wobei das biologische Fettgewebe mikrofragmentiertes menschliches Fettgewebe ist.

3. Verfahren nach Anspruch 1 oder 2, wobei die Lösungen von Trehalose und DMSO Lösungen in Wasser oder in physiologischer Salzlösung sind.

4. Verfahren nach einem der vorangehenden Ansprüche, wobei die Lösungen von Trehalose und DMSO alle die gleiche Konzentration von Trehalose und zu Null hin abnehmende Konzentrationen von DMSO aufweisen.

5. Verfahren nach einem der vorhergehenden Ansprüche, wobei die Konzentration von Trehalose und die Konzentration von DMSO im Bereich von 0,05 M bis 0,4 M, vorzugsweise von 0,1 M bis 0,3 M liegen.

6. Verfahren nach einem der vorangehenden Ansprüche, wobei die Auftauphase mindestens drei aufeinanderfolgende Waschungen des Gewebes mit Lösungen von Trehalose und DMSO enthalten.

7. Verfahren nach einem der vorhergehenden Ansprüche, wobei die Auftauphase drei aufeinanderfolgende Waschungen des Gewebes mit den folgenden Lösungen enthält:
- Lösung 1: 0,3 M DMSO + 0,2 M Trehalose
- Lösung 2: 0,1 M DMSO + 0,2 M Trehalose
- Lösung 3: 0,2 M Trehalose.

8. Verfahren nach einem der vorangehenden Ansprüche, wobei die Gefrierphase an dem Gewebe durchgeführt wird, dem ein kryoprotektives Mittel, wie eine Lösung von Trehalose und DMSO in Wasser oder in physiologischer Salzlösung, zugesetzt wurde.

9. Verfahren nach einem der vorangehenden Ansprüche, wobei die Gefrierphase durchgeführt wird, indem das Gewebe in einen geeigneten Behälter innerhalb eines Schnellkühlers mit einer Temperaturabsenkungsprogrammierung bis auf -180 °C gelegt wird, wobei dann der Behälter unter Flüssigstickstoff transferiert wird.

10. Kit zur Durchführung des Verfahrens nach den Ansprüchen 1 - 9, enthaltend mehrere Behälter, die in der Lage sind, die Probe von biologischem Fettgewebe in der Auftauphase für ihre Waschung aufzunehmen, und mehrere gebrauchsfertige Waschlösungen von Trehalose und DMSO bei einer zu Null hin abnehmenden Konzentration von DMSO.

11. Verwendung des Kits nach Anspruch 10 zum Auftauen von Proben menschlichen Fettgewebes, das die aus der Kryokonservierung stammen und zur Transplantation in der kosmetischen Chirurgie und der regenerativen Medizin bestimmt sind.

## Revendications

1. Un procédé pour la cryoconservation d'un échantillon de tissu adipeux humain préalablement prélevé sur un sujet, comprenant une phase de congélation dudit tissu, une phase de stockage du tissu congelé pendant un certain temps, et une phase de décongélation du tissu, ledit procédé étant **caractérisé en ce que** ladite phase de décongélation est réalisée par lavages successifs dudit tissu avec des solutions de tréhalose et de diméthylsulfoxyde (DMSO), à concentration décroissante jusqu'à zéro en DMSO, le dernier lavage étant réalisé avec une solution de tréhalose seul.

2. Le procédé selon la revendication 1, dans lequel ledit tissu biologique est un tissu adipeux humain micro-fragmenté.

3. Le procédé selon l'une des revendications 1 et 2, dans lequel lesdites solutions de tréhalose et de DMSO sont des solutions dans de l'eau ou dans une solution saline physiologique.

4. Le procédé selon l'une quelconque des revendications précédentes, dans lequel lesdites solutions de tréhalose et de DMSO ont toutes la même concentration de tréhalose et des concentrations décroissantes jusqu'à zéro de DMSO.

5. Le procédé selon l'une quelconque des revendications précédentes, dans lequel ladite concentration de tréhalose et ladite concentration de DMSO est comprise entre 0,05 M et 0,4 M, de préférence entre 0,1 M et 0,3 M.

6. Le procédé selon l'une quelconque des revendications précédentes, dans lequel ladite phase de décongélation comprend au moins trois lavages successifs dudit tissu avec lesdites solutions de tréhalose et de DMSO.

7. Le procédé selon l'une quelconque des revendications précédentes, dans lequel ladite phase de décongélation comprend trois lavages successifs dudit tissu avec les solutions suivantes :
- Solution 1 : 0,3 M de DMSO + 0,2 M de tréhalose
- Solution 2 : 0,1 M de DMSO + 0,2 M de tréhalose
- Solution 3 : 0,2 M de tréhalose.

8. Le procédé selon l'une quelconque des revendications précédentes, dans lequel ladite phase de congélation est réalisée sur ledit tissu additionné d'un agent cryoprotecteur, telle qu'une solution de tréhalose et de DMSO dans de l'eau ou dans une solution saline physiologique.

9. Le procédé selon l'une quelconque des revendications précédentes, dans lequel ladite phase de congélation est réalisée en plaçant le tissu dans un récipient approprié à l'intérieur d'une cellule de refroidissement rapide ayant une programmation d'abaissement de température jusqu'à -180°C, puis en transférant ledit récipient sous azote liquide.

10. Un kit pour la mise en oeuvre du procédé selon l'une quelconque des revendications 1 à 9, comprenant plusieurs récipients capables d'héberger ledit échantillon de tissu biologique en phase de décongélation pour son lavage, et plusieurs solutions de lavage prêtes à l'emploi de tréhalose et de DMSO, à concentration de DMSO décroissante jusqu'à zéro.

11. Utilisation du kit selon la revendication 10, pour la décongélation d'échantillons de tissu adipeux humain provenant de la cryoconservation et destinés à la greffe en chirurgie esthétique et en médecine régénératrice.
